(19) Europäisches Patentamt
European Patent Office
Office européen des brevets

(11) **EP 0 402 724 B2**

(12) **NEUE EUROPÄISCHE PATENTSCHRIFT**

(45) Veröffentlichungstag und Bekanntmachung des Hinweises auf die Entscheidung über den Einspruch:
**09.05.2001   Patentblatt 2001/19**

(45) Hinweis auf die Patenterteilung:
**14.02.1996   Patentblatt 1996/07**

(21) Anmeldenummer: **90110531.2**

(22) Anmeldetag: **02.06.1990**

(51) Int Cl.[7]: **C08B 31/12, A61K 31/718**

(54) **Hydroxethylstärke als Plasma-expander und Verfahren zu ihrer Herstellung**

Hydroxyethyl starch as plasma expander and process for its preparation

Hydroxyéthylamidon comme diluant du plasma et son procédé de préparation

(84) Benannte Vertragsstaaten:
**AT BE CH DE ES FR GB GR IT LI LU NL SE**

(30) Priorität: **16.06.1989   DE 3919729**

(43) Veröffentlichungstag der Anmeldung:
**19.12.1990   Patentblatt 1990/51**

(73) Patentinhaber: **Fresenius AG**
**61350 Bad Homburg (DE)**

(72) Erfinder:
• **Sommermeyer, Klaus, Dr.**
**D-6365 Rosbach v.d.H (DE)**
• **Cech, Franz, Dr.**
**D-6365 Rosbach v.d.H (DE)**
• **Weidler, Burghard, Dr.**
**D-6365 Rosbach 1 (DE)**
• **Henning, Klaus, Dr.**
**D-6390 Usingen 1 (DE)**

(74) Vertreter: **Luderschmidt, Schüler & Partner GbR Patentanwälte,**
**John-F.-Kennedy-Strasse 4**
**65189 Wiesbaden (DE)**

(56) Entgegenhaltungen:
DE-A- 2 814 032          DE-A- 3 313 600
DE-A- 4 310 974          GB-A- 1 395 777
US-A- 4 016 354

• DIE STÄRKE, Band 34, Nr. 2, 1982, Seiten 65-68, Verlag Chemie GmbH, Weinheim, DE; S.I. EL-HINNAWY et al.: "Preparation and evaluation of hydroxyethyl starch"

• CHEMICAL ABSTRACTS, Band 93, Nr. 23, 8. Dezember 1980, Seite 360, Zusammenfassung Nr. 218714c, Columbus, Ohio, US; J.M. MISHLER et al.: "Changes in the molecular size distribution and post-transfusion survival of hydroxyethylstarch 350/0.60 as influenced by a lower degree of hydroxyethylation: a study n mormal man", & J.CLIN. PATHOL. 1980, 33(9), 880-4

• DIE STÄRKE, Band 29, Nr. 12, 1977, Verlag Chemie, GmbH, Weinheim, DE; H.G. MERKUS et al.: "Substituent distribution in hydroxyethyl starch"

• K. Sommermeyer et al., Krankenhauspharmazie 8 (1987) 271-278

• H. Förster, Beitr. Anaesth. Intensivmed. 26 (1988) 27-42

• M. Yoshida et al., "Die Stärke" 25.Jahrg., Nr. 11, S. 373-376 (1973)

• H. Förster, Beitr. Anaesth, Intensivmed, 26, 1988, 27-42

• M. Yoshida und T. Kishikawa, Starch/Stärke, 36, 1984, 167-169

• M. Yoshida et al, Starch/Stärke, 36, 1984, 209,212

• J.M. Mishler, Int, J. Clinical Pharm., 18(2), 1980, 67-72

• C.Y. Sum et al, J. of Chromatography, 254, 1983, 187-194

• F. Asskali, Beitr. Anaesth. Intensivmed., 26, 1988, 43-53

• H.P. Ferber, Habilitationsschrift der J.W.-Goethe Universität, Frankfurt/a.M., 1985, 14-22, 32, 33 und 198

• Rote Liste 1988, 51 352, 51 353

• W. Banks et al, Br. J. Pharmac., 47, 1973, 172-178

• H. Lutz, Plasmaersatzmittel, G.Thieme Verlag, Stuttgart, 1980, 3. Aufl. 86

- **W.L. Thompson, Develop. biol. Standard, 48, 1981, 259-266**
- **Roche Lexikon Medizin, 1984/1987, Urban & Schwarzenberg, 1363**
- **Römpps Chemie-Lexikon, 1990, 9. Aufl., 1252**
- **ABC Chemie, 1965, Bd. 1, Verlag H. Deutsch, Frankfurt/M., 124, 766**
- **H.G. Elias, "Makromoleküle, 277-280**
- **Rote Liste 1996, 52 245-52 247**
- **N. Gretz et al, Nephron, 61, 1992, 120**
- **Vergleichsversuche eingereicht am 8.6.94 (Eine Abbildungsseite)**
- **Vergleichsversuche von Dr. Förster eingereicht am 7.3.95**

Bemerkungen:

Die Akte enthält technische Angaben, die nach dem Eingang der Anmeldung eingereicht wurden und die nicht in dieser Patentschrift enthalten sind.

**Beschreibung**

Kolloidale Plasmaersatzmittel sind heute im Bereich des Volumenersatzes (z.B. hämorrhagischer Schock) oder der Hämodilution (z. B. arterielle Verschlußkrankheit, Fontaine II B, III) nicht mehr wegzudenken. Von den körperfremden Plasmaersatzmitteln (Stärke, Gelatine, Dextran) hat die Hydroxyethylstärke (HES) in den letzten Jahren die größte Akzeptanz bei beiden Indikationen gefunden.

Für die gute Akzeptanz der Hydroxyethylstärke im Bereich des Volumenersatzes und der Hämodilution sind die geringe Störung der Gerinnung und die deutlich verminderte Inzidenz schwerer anaphylaktoider Reaktionen im Vergleich zu Dextran verantwortlich. Zudem konnte gezeigt werden, daß die Volumenwirksamkeit der Hydroxyethylstärke je nach Indikation als ausreichend bis gut bezeichnet werden kann, wobei durch die verschiedenen bekannten Hydroxyethylstärke-Präparate, die sich in Molekulargewicht und Substitutionsgrad unterscheiden, eine differenzierte Therapie je nach Zustand des Patienten möglich wird. Besonders positiv wird hierbei der geringe kolloidosmotische Druck von Stärkelösungen im Vergleich zu Dextranen bewertet. Bezogen auf die Niere beinhaltet die niedrigere Urinviskosität ein geringeres Risiko der renalen Funktionsminderung. Im Bereich der Hämodilution konnte als therapeutisch wirksames Prinzip der HES-induzierten rheologischen Verbesserung, neben der Senkung des Hämatokrits vor allem die Reduktion der Plasmaviskosität herausgearbeitet werden. Hierdurch ergeben sich therapeutische Vorteile gegenüber anderen körperfremden Plasmaersatzmitteln.

Bereits bekannte Hydroxyethylstärken, die als Plasmaexpander eingesetzt werden, weisen verschiedene Molekulargewichte Mw sowie Substitutionsgrade MS und DS als auch verschiedene Substitutionsmuster auf.

Bedingt durch den Einsatz des natürlichen Ausgangsrohrstoffes Amylopektin sowie durch das Herstellungsverfahren, bei dem im gewissen Umfang eine Spaltung der Polymerketten notwendig ist, liegt Hydroxyethylstärke nicht als molekulareinheitliche Substanz mit definiertem Molekulargewicht vor, sondern als Gemisch von Molekülen unterschiedlicher Größe, die auch verschieden durch Hydroxyethylgruppen substituiert sind. Die Charakterisierung solcher Gemische bedarf der Zuhilfenahme statistisch gemittelter Größen (vgl. K. Sommermeyer et. al., "Klinisch verwendete Hydroxyethylstärke: Physikalisch-chemische Charakterisierung", Krankenhauspharmazie, 271 (1987)). Zur Kennzeichnung des durchschnittlichen Molekulargewichts dient daher das gemittelte Molekulargewicht $M_w$. Die allgemeine Definition dieses Mittelwerts lautet:

$$\overline{M_w} = \frac{\sum\limits_{i} N_i \cdot M_i^w}{\sum\limits_{i} N_i \cdot M^{w-1}}$$

Für die Erfassung der Substitution durch Hydroxyethylgruppen existieren zwei unterschiedlich definierte Substitutionsgrade.

Der Substitutionsgrad MS (molar substitution) ist definiert als die durchschnittliche Anzahl von Hydroxyethylgruppen pro Anhydroglucoseeinheit. Er wird ermittelt aus der Gesamtanzahl der Hydroxyethylgruppen in einer Probe, beispielsweise nach Morgan, durch Atherspaltung und anschließender quantitativer Bestimmung von Ethyliodid und Ethylen, die hierbei gebildet werden.

Hingegen ist der Substitutionsgrad DS (degree of substitution) definiert als der Anteil der substituierten Anhydroglucoseeinheiten aller Anhydroglucoseeinheiten. Ihn kann man bestimmen aus der gemessenen Menge der unsubstituierten Glucose nach Hydrolyse einer Probe. Aus diesen Definitionen ergibt sich, daß MS > DS. Für den Fall, daß nur Monosubstitution vorliegt, also jede substituierte Anhydroglucoseeinheit nur eine Hydroxyethylgruppe trägt, ist MS = DS.

Es ist bekannt, daß α-Amylase Hydroxyethylstärken in dem Sinne abbaut, daß nur glycosidische Bindungen unsubstituierter Anhydroglucoseeinheiten gespalten werden. Es ist weiterhin bekannt, daß mit steigendem Substitutionsgrad MS bzw. DS die Eliminierung von Hydroxyethylstärken aus dem Plasma verlangsamt wird.

Des weiteren ist bekannt, daß bei gleichem MS, DS und gleicher Molekulargewichtsverteilung überwiegend in 6-Position substituierte Stärken schneller eliminiert werden als überwiegend in 2-Position substituierte Stärken.

Insofern gelangten für pharmazeutische Zwecke ausschließlich Hydroxyethylstärken zur Anwendung, die ein niedriges C2/C6-Verhältnis aufweisen bzw. hoch substituiert sind.

So beschreibt die GB-PS 1,395,777 überwiegend in 6-Position substituierte Hydroxyethylstärken entsprechend einem Verhältnis C2/C6 von 0,5 bis 2,0. Diese Hydroxyethylstärken werden durch Reaktion von Wachsmaisstärke mit Ethylenoxid mit Alkali im Überschuß hergestellt.

In der DE-OS 28 14 032 wird ein Verfahren zur Herstellung von als Blutplasmaexpander geeigneter Hydroxylstärke beschrieben, wobei die Stärke alkalisch hydroxyethyliert, dann das Reaktionsgemisch neutralisiert und die gebildete Hydroxyethylstärke aus dem Reaktionsgemisch mit einem Lösungsmittel, wie Dimethylformamid, in dem die durch die Neutralisation entstandenen Salze nur wenig bis gar nicht löslich sind, extrahiert wird. Die erhaltene Hydroxyethylstärke weist ein molares Verhältnis von 2-O-Hydroxyethylanhydroglucose zu 6-O-Hydroxyethylanhydroglucose von etwa 1 auf.

Gemäß dem in der DE-OS 33 13 600 beschriebenen Verfahren zur Herstellung von Plasmastreckmitteln auf Stärkebasis, bei dem der Abbauschritt der an Amylopektin reichen Stärke zumindest teilweise enzymatisch durchgeführt wird, wird der Abbau der Stärke bis zu einem Molekulargewicht von 40.000 bis 1.000.000 Dalton, insbesondere von 200.000 bis 450.000 Dalton, und die Veretherung bis zu einem Substitutionsgrad (MS) von 0,1 bis 0,8 bzw. 0,5 bis 0,8, insbesondere von 0,5 bis 0,7 (vgl. Seite 8, Absatz 3), durchgeführt. Das Verhältnis der Substitution an C2 gegenüber der Substitution an C6 ist niedrig (vgl. Seite 5, Absatz 2).

Die genannten Hydroxyethylstärken haben den Nachteil, daß sie keine vollständige Abbaubarkeit aus dem Plasma innerhalb einer Zeitspanne von ca. 6-12 Stunden gewährleisten und außerdem, aufgrund ihres hohen Substitutionsgrades MS (MS > 0,5) die Gefahr in sich bergen, daß bei den üblichen Wiederholungsinfusionen über längere Zeiträume eine Akkumulation von schwer eliminierbaren Anteilen im Serum und im Gewebe entsteht. Durch diese Langzeitspeicherung kann es zu allergischen Reaktionen, wie z.B. Nesselfieber etc., kommen.

Aufgabe der vorliegenden Erfindung ist es daher, eine Hydroxyethylstärke zur Verfügung zu stellen, die innerhalb einer physiologisch vernünftigen Zeit restlos abbaubar ist.

Eine weitere Aufgabe besteht darin, eine HES zur Verfügung zu stellen, das dennoch aufgrund der Wahl eines geeigneten MS- bzw. DS-Wertes und des Molekulargewichts ein steuerbares Eliminationsverhalten aufweist.

Ausgangsprodukte für die Gewinnung von Hydroxyethylstärke sind solche Stärken, die einen hohen Gehalt an Amylopektin, der hochverzweigten Komponente von Stärke, aufweisen, insbesondere Kartoffelstärke, Wachsmaisstärke, Sorghumstärke oder wachsartige Reisstärke.

Zur groben Voreinstellung des beabsichtigten Molekulargewichts werden diese Stärken einer hydrolytischen Abbaureaktion unterworfen. Dabei wird das Molekulargewicht von etwa 20.000.000 Dalton auf mehrere Millionen Dalton reduziert.

Bei der anschließenden alkalischen Hydroxyethylierung mit bekannten Hydroxyethylierungsmitteln ist die Einführung einer Hydroxyethylgruppe in Position 2, 3 und 6 der Anhydroglucoseeinheit möglich. Disubstituierte Einheiten, wie 2,3-Dihydroxyethylanhydroglucose, 2,6-Dihydroxyethylanhydroglucose werden dabei mit geringerer Wahrscheinlichkeit bei der Synthese gebildet. Die Reaktivität der einzelnen Hydroxygruppen in der unsubstituierten Anhydroglucoseeinheit gegenüber Hydroxyethylierung ist je nach Reaktionsbedingungen unterschiedlich. Innerhalb gewisser Grenzen ist dadurch das Substitutionsmuster, also die einzelnen, unterschiedlich substituierten Anhydroglucosen, die statistisch auf die einzelnen Polymermoleküle verteilt sind, beeinflußbar. Vorteilhaft werden überwiegend die 2- und die 6-Position hydroxyethyliert, wobei die 6-Position aufgrund ihrer leichteren Zugänglichkeit bevorzugt substituiert wird.

Das Ziel der vorliegenden Erfindung, nämlich die Darstellung einer innerhalb einer physiologisch vernünftigen Zeit restlos abbaubaren Hydroxyethylstärke, die auf der anderen Seite dennoch ein steuerbares Eliminationsverhalten aufweist, wird erreicht durch eine überwiegend in 2-Position substituierte Stärke, die möglichst homogen substituiert ist, wobei MS ungefähr gleich DS ist.

Die überwiegende 2-Substitution macht die Hydroxyethylstärke relativ schwierig abbaubar für α-Amylase. Es ist von Vorteil, daß möglichst keine innerhalb der Polymermoleküle hintereinander substituierten Anhydroglucoseeinheiten auftreten, um die restlose Abbaubarkeit zu gewährleisten.

Dies kann dadurch erreicht werden, daß man entsprechend niedrig substituiert, was es erlaubt, die Moleküle statistisch im Sinne einer über die gesamten Moleküle verteilten Substitution zu derivatisieren. Dadurch erhält man substituierte Anhydroglucosen in relativ großem Abstand zueinander, wodurch der durch die überwiegende 2-Substitution bedingte Effekt der Verlangsamung des α-Amylaseabbaus kompensiert und eine Steuerbarkeit der Abbaugeschwindigkeit erreicht werden kann.

Es wurde gefunden, daß Hydroxyethylstärken, die außergewöhnlich niedrig substituiert sind (MS ≤ 0,5) und die ein hohes Verhältnis der Substitution an C2 zur Substitution an C6 der Anhydroglucoseeinheiten aufweisen, innerhalb der ersten Stunden der Infusion rasch und vollständig aus dem menschlichen Körper eliminiert werden.

Weiterhin wurde gefunden, daß solche Hydroxyethylstärken trotz der niedrigen Substitution, entgegen der Meinung der Fachwelt, eine ausreichend hohe Löslichkeit in wässrigem Medium besitzen, so daß die Lösungen auch über längere Zeiträume stabil sind und sich keine Agglomerate bzw. Gele bilden, die den weiteren Einsatz als Plasmaexpander-Lösung verbieten würden. Hydroxyethylstärken mit den oben beschriebenen Charakteristika vereinigen deshalb die generellen Vorteile von Hydroxyethylstärke gegenüber anderen Plasmaexpander-Typen, wie Gelatine oder Dextran, und vermeiden die Nachteile der bisher bekannten Hydroxyethylstärke-Typen, die zu den beschriebenen Indikationen eingesetzt werden.

Die Erfindung betrifft hydroxyethylstärke zum Einsatz als Plasmaexpander, erhältlich durch hydrolytischen Vor-Abbau einer an Amylopektin reichen Stärke, partielle Hydroxyethylierung bis zu einem bestimmten Substitutionsgrad in Gegenwart von Alkali und anschließenden hydrolytischen Abbau auf ein bestimmtes Molekulargewicht, dadurch gekennzeichnet, daß

sie ein mittleres Molekulargewicht Mw von 80.000 bis 300.000 und einen Substitutionsgrad MS Von 0,2 - 0,4 aufweist, das Verhältnis der Substitution an C2 zur Substitution an C6 der Anhydroglucoseeinheiten 8 - 20 beträgt und der Substitutionsgrad DS im Bereich von 0,15 bis 0,40 liegt.

Hydroxyethylstärken mit den genannten Eigenschaften können erhalten werden mit Hilfe eines Verfahrens, das im wesentlichen folgende Schritte enthält:

a) Vorextrahieren der verwendeten Stärke mit Methanol zur Entfernung von Pflanzenfarbstoffen und Blockierung von reaktiven Gruppen. So werden z. B. reaktive Aldehyd-Gruppierungen teilweise durch Acetalbildung inaktiviert.

b) Methanolische Hydrolyse zur Grobeinstellung des Molekulargewichts mit einer 20 - 40%igen, bevorzugt 30%igen methanolischen Suspension der Stärke mit 1 % HCl, wobei diese für 2 - 4h, bevorzugt 3h, auf 30 - 50°C, bevorzugt 40°C, gehalten wird. Das Ende der Reaktion wird dabei durch Neutralisation mit 1 N NaOH und anschließendes Abkühlen auf Raumtemperatur erreicht. Anschließend wird die Suspension chloridfrei gewaschen.

c) Alkaliwäsche zur Proteinextraktion, wobei eine 30 - 50%ige, bevorzugt 40%ige Suspension in 0,1 N NaOH hergestellt wird und diese 1 - 3 h, bevorzugt 2h, bei 30 - 50°C, bevorzugt 40°C, gehalten wird. Anschließend wird die Prozedur bei Raumtemperatur wiederholt.

d) Hydroxyethylierung mit einem Hydroxyethylierungsmittel, z.B. Ethylenoxid, und in einer besonders bevorzugten Ausführungsform, 2-Chlorethanol, wobei das molare Verhältnis von vorbehandelter Stärke zu Hydroxyethylierungsmittel dem gewünschten Substitutionsgrad angepaßt wird. Die Stärke wird in 20 - 40%iger, bevorzugt 30%iger Suspension in 1 N NaOH 2h bei 30 - 50°C, bevorzugt 40°C unter Stickstoff gelöst. Innerhalb 6 - 10 Stdn., bevorzugt 7 - 8 Stdn., wird das Hydroxyethylierungsmittel bei Raumtemperatur zugetropft, wobei durch Zugabe von 10 N NaOH verhindert wird, daß der pH-Wert unter 12 absinkt. Anschließend wird mit 10%iger HCl neutralisiert.

e) Die Lösung wird auf 40 - 70°C, bevorzugt 60°C, erwärmt, mit 0,2 % HCl versetzt, und die Hydrolyse viskosimetrisch verfolgt. Die Reaktion wird durch Neutralisation mit NaOH und Abkühlen auf Raumtemperatur beendet.

f) Reinigung durch Filtration über ein Tiefenfilter und Ultrafiltration über ein Hohlfaser-Modul mit einer Trenngrenze von ca. 30.000 Dalton.

g) Sprühtrocknung der Endprodukte in an sich bekannter Weise.

Die erfindungsgemäßen Hydroxyethylstärken sind auch geeignet als Kohlenhydratkomponente bei der enteralen Ernährung von Diabetikern, da bezüglich der Abbaubarkeit dieselben Überlegungen gelten.

Die Erfindung wird im folgenden anhand eines Beispiels näher erläutert.

500g Wachsmaisstärke werden in einem Liter trockenem Methanol aufgeschlämmt und zum Sieden erhitzt. Nach dem Abkühlen wird das Methanol abgesaugt und die Stärke mit Wasser nachgewaschen. Der Waschvorgang wird einmal wiederholt.

Die Stärke mit einem Restfeuchtegehalt von 28,13 % wird in 30-%iger methanolischer Suspension mit 1 % HCl 3 Stunden bei 40°C hydrolysiert. Die Reaktion wird durch Neutralisation mit 1 N NaOH in Methanol und Abkühlen auf Raumtemperatur gestoppt. Nach dem Absaugen zeigt die Stärke einen Restfeuchtegehalt von 16,12 % und ein mittleres Molekulargewicht von 900.000.

Die Stärke wird in einem Liter $H_2O$ aufgeschlämmt, gerührt, abgesaugt und chloridfrei gewaschen. Nach dem Trockensaugen hat die Stärke einen Restfeuchtegehalt von 51,29 %.

Anschließend wird die Stärke in 40 %-iger Suspension in 0,1 N NaOH 2 Stunden bei 40°C gerührt, wieder auf Raumtemperatur abgekühlt und trockengesaugt (Restfeuchtegehalt 48,60 %). Der Vorgang wird bei Raumtemperatur einmal wiederholt.

418,0 g (2,58 Mol) der vorbehandelten Stärke werden in 30 %-iger Suspension in 1 N NaOH bei 40°C unter Stickstoff gelöst. Innerhalb von 7 - 8 Stdn. werden bei 20°C 51,9 ml (0,77 Mol) 2-Chlorethanol zugetropft. Durch Zugabe von NaOH wird ein Absinken des pH-Wertes unter 12 vermieden. Danach wird mit 10 %-iger HCl neutralisiert.

Die Lösung wird nach einer 1:1-Verdünnung mit Wasser über einen Tiefenfilter (Seitz T750) filtriert.

Danach wird auf 60°C erwärmt, mit 25 %iger HCl auf eine HCl-Konzentration von 0,2 eingestellt und 4 Stdn. hydrolysiert. Die Lösung wird durch Zugabe von Natronlauge auf pH 6,0 neutralisiert und auf Raumtemperatur abgekühlt. Anschließend wird über ein Seitz EKS-Filter filtriert.

Die klare Lösung wird nun über ein Hohlfaser-Modul mit einer Trenngrenze von ca. 30.000 Dalton ultrafiltriert und das verbliebene Retentat sprühgetrocknet.

Man erhält eine Hydroxyethylstärke mit einem mittleren Molekulargewicht von 234.000 und einem molaren Substitutionsgrad von 0,26. Das C2/C6-Verhältnis beträgt 9,34.

Die auf diese Weise hergestellte Hydroxyethylstärke weist folgendes, durch vollständige Hydrolyse von HES und anschließende Bestimmung von Glucose und deren Hydroxyethylderivate über Trimethylsilylierung bestimmbares Substitutionsmuster (Flächenprozente) auf:

| Glukose | 81,42 % |
|---|---|
| 2-0-Hydroxyethylglucose | 12,42 % |
| 3-0-Hydroxyethylglucose | 2,70 % |
| 6-0-Hydroxyethylglucose | 1,33 % |
| 2,2-0-Dihydroxyethylglucose | 0,21 % |
| 2,3-0-Dihydroxyethylglucose | 0,51 % |
| 2,6-0-Dihydroxyethylglucose | 0,17 % |
| 3,3-0-Dihydroxyethylglucose | 0,10 % |
| 3,6-0-Dihydroxyethylglucose | 0,05 % |

## Patentansprüche

### Patentansprüche für folgende Vertragsstaaten : AT, BE, CH, LI, DE, FR, GB, GR, IT, LU, NL, SE

1.  Hydroxyethylstärke zum Einsatz als Plasmaexpander, erhältlich durch hydrolytischen Vor-Abbau einer an Amylopektin reichen Stärke, partielle Hydroxyethylierung bis zu einem bestimmten Substitutionsgrad in Gegenwart von Alkali und anschließendem hydrolytischen Abbau auf ein bestimmtes Molekulargewicht, dadurch gekennzeichnet, daß

    sie ein mittleres Molekulargewicht $M_w$ von 80.000 bis 300.000 und einen Substitutionsgrad MS von 0,2 bis 0,4 aufweist,

    das Verhältnis der Substitution an $C_2$ zur Substitution an $C_6$ der Anhydroglucoseeinheiten 8 bis 20 beträgt und

    der Substitutionsgrad DS im Bereich von 0,15 bis 0,40 liegt.

2.  Hydroxyethylstärke nach Anspruch 1, dadurch gekennzeichnet, daß sie ein mittleres Molekulargewicht $M_w$ von 100.000 bis 300.000 und einen Substitutionsgrad MS von 0,25 bis 0,35 aufweist, das Verhältnis der Substitution an $C_2$ zur Substitution an $C_6$ der Anhydroglucoseeinheiten 8 bis 20 beträgt und der Substitutionsgrad DS im Bereich von 0,2 bis 0,35 liegt.

3.  Verfahren zur Herstellung von Hydroxyethylstärke nach Anspruch 1, bei dem

    a) Stärke die einen Gehalt an Amylopektin von > 95% aufweist, mit Methanol vorextrahiert wird,

    b) die Stärke durch Säurehydrolyse auf ein bestimmtes mittleres Molekulargewicht gebracht wird,

    c) die Stärke einer Alkaliwäsche unterworfen wird,

    d) die Stärke mittels eines Hydroxyethylierungsmittels unter alkalischen Bedingungen hydroxyethyliert wird,

    e) das Molekulargewicht durch Säurehydrolyse genau eingestellt wird,

    f) die so erhaltene Hydroxyethylstärke gereinigt und

    g) sprühgetrocknet wird,

dadurch gekennzeichnet, daß als Hydroxyethylierungsmittel 2-Chlorethanol verwendet wird und die Hydroxyethylierung unter alkalischen Bedingungen bei Raumtemperatur durchgeführt wird.

4.  Verfahren nach Anspruch 3, dadurch gekennzeichnet, daß der pH-Wert bei einem Wert von etwa 12 während der Hydroxyethylierung gehalten wird.

5.  Verfahren nach Anspruch 3 oder 4, dadurch gekennzeichnet, daß die Temperatur bei einem Wert von etwa 20 bis 25°C gehalten wird.

6.  Verfahren nach einem der Ansprüche 3 bis 5, dadurch gekennzeichnet, daß die Hydroxyethylstärke durch Filtration und Ultrafiltration gereinigt wird.

7.  Verwendung von Hydroxyethylstärke nach mindestens einem der vorhergehenden Ansprüche bei der Herstellung eines kolloidalen Plasmaersatzmittels.

### Patentansprüche für folgenden Vertragsstaat : ES

1.  Verwendung von Hydroxyethylstärke, erhältlich durch hydrolytischen Vor-Abbau einer an Amylopektin reichen Stärke, partielle Hydroxyethylierung bis zu einem bestimmten Substitutionsgrad in Gegenwart von Alkali und anschließenden hydrolytischen Abbau auf ein bestimmtes Molekulargewicht, bei der Herstellung eines kolloidalen Plasmaersatzmittels wobei die Hydroxyethylstärke ein mittleres Molekulargewicht $M_w$ von 80.000 bis 300.000 und einen Substitutionsgrad MS von 0,2 bis 0,4 aufweist, das Verhältnis der Substitution an C2 zur Substitution an C6 der Anhydroglucoseeinheiten 8 bis 20 beträgt und der Substitutionsgrad DS im Bereich von 0,15 bis 0,40 liegt.

**2.** Verwendung nach Anspruch 1, dadurch gekennzeichnet, daß die Hydroxyethylstärke ein mittleres Molekulargewicht MS von 100.000 bis 300.000 und einen Substitutionsgrad MS von 0,25 bis 0,35 aufweist, das Verhältnis der Substitution an C2 zur Substitution an C6 der Anhydroglucoseeinheiten 8 bis 20 beträgt und der Substitutionsgrad DS im Bereich von 0,2 bis 0,35 liegt.

**3.** Verfahren zur Herstellung von Hydroxyethylstärke nach Anspruch 1 oder 2, bei dem

a) Stärke, die einen Gehalt an Amylopektin von > 95 % aufweist, mit Methanol vorextrahiert wird,

b) die Stärke durch Säurehydrolyse auf ein geeignetes mittleres Molekulargewicht gebracht wird,

c) die Stärke einer Alkaliwäsche unterworfen wird,

d) die Stärke mittels eines Hydroxyethylierungsmittels unter alkalischen Bedingungen hydroxyethyliert wird,

e) das Molekulargewicht durch Säurehydrolyse genau eingestellt wird,

f) die so erhaltene Hydroxyethylstärke gereinigt und

g) sprühgetrocknet wird,

dadurch gekennzeichnet, daß als Hydroxyethylierungsmittel 2-Chlorethanol verwendet wird und die Hydroxyethylierung unter alkalischen Bedingungen bei Raumtemperatur durchgeführt wird.

**4.** Verfahren nach Anspruchs 3, dadurch gekennzeichnet, daß der pH-Wert bei einem Wert von etwa 12 während der Hydroxyethylierung gehalten wird.

**5.** Verfahren nach Anspruch 3 oder 4, dadurch gekennzeichnet, daß die Temperatur bei einem Wert von etwa 20 bis 25°C gehalten wird.

**6.** Verfahren nach einem der Ansprüche 3 bis 5, dadurch gekennzeichnet, daß die Hydroxyethylstärke durch Filtration und Ultrafiltration gereinigt wird.

**Claims**

**Claims for the following Contracting States : AT, BE, CH, LI, DE, FR, GB, GR, IT, LU, NL, SE**

**1.** Use of hydroxyethyl starch, obtainable by hydrolytic pre-degradation of a starch rich in amylopectin, partial hydroxyethylation up to a certain degree of substitution in the presence of alkali and subsequent hydrolytic degradation to a certain molecular weight, in the production of a colloidal plasma substitute, wherein the hydroxyethyl starch has a mean molecular weight $M_w$ of 80,000 to 300,000 and a degree of substitution MS of 0.2 to 0.4, the ratio of the substitution of $C_2$ to the substitution of $C_6$ of the anhydroglucose units is 8 to 20 and the degree of substitution DS lies in the range from 0.15 to 0.40.

**2.** Use according to claim 1, characterised in that the hydroxyethyl starch has a mean molecular weight MS of 100,000 to 300,000 and a degree of substitution MS of 0.25 - 0.35, the ratio of the substitution of $C_2$ to the substitution of $C_6$ of the anhydroglucose units is 8 to 20 and the degree of substitution DS lies in the range from 0.2 to 0.35.

**3.** Process for the preparation of hydroxyethyl starch according to claim 1 or claim 2, wherein

a) starch having a content of amylopectin of > 95% is preextracted with methanol,

b) the starch is brought by acid hydrolysis to a certain mean molecular weight,

c) the starch is subjected to an alkali wash,

d) the starch is hydroxyethylated by means of a hydroxyethylation agent under alkaline conditions,

e) the molecular weight is set precisely by acid hydrolysis,

f) the hydroxyethyl starch thus obtained is purified and

g) spray-dried,

characterised in that 2-chloroethanol is used as hydroxyethylation agent and hydroxyethylation is carried out under alkaline conditions at room temperature.

**4.** Process according to claim 3, characterised in that the pH value is kept at a value of about 12 during hydroxyethylation.

5. Process according to claim 3 or claim 4, characterised in that the temperature is kept at a value of about 20 to 25°C.

6. Process according to any one of claims 3 to 5, characterised in that the hydroxyethyl starch is purified by filtration and ultrafiltration.

7. Use of hydroxyethyl starch according to at least one of the preceding claims in the production of a colloidal plasma substitute.

**Claims for the following Contracting State : ES**

1. Hydroxyethyl starch for use as a plasma expander, obtainable by hydrolytic pre-degradation of a starch rich in amylopectin, partial hydroxyethylation up to a certain degree of substitution in the presence of alkali and subsequent hydrolytic degradation to a certain molecular weight, characterised in that

   it has a mean molecular weight $M_w$ of 80,000 to 300,000 and a degree of substitution MS of 0.2 to 0.4,

   the ratio of the substitution of $C_2$ to the substitution of $C_6$ of the anhydroglucose units is 8 to 20, and

   the degree of substitution DS lies in the range from 0.15 to 0.40.

2. Hydroxyethyl starch according to claim 1, characterised in that it has a mean molecular weight $M_w$ of 100,000 to 300,000 and a degree of substitution MS of 0.25 to 0.35, the ratio of the substitution of $C_2$ to the substitution of $C_6$ of the anhydroglucose units is 8 to 20 and the degree of substitution DS lies in the range from 0.2 to 0.35.

3. Process for the preparation of hydroxyethyl starch according to claim 1, wherein

   a) starch having a content of amylopectin of > 95% is preextracted with methanol,

   b) the starch is brought by acid hydrolysis to a suitable mean molecular weight,

   c) the starch is subjected to an alkali wash,

   d) the starch is hydroxyethylated by means of a hydroxyethylation agent under alkaline conditions,

   e) the molecular weight is set precisely by acid hydrolysis,

   f) the hydroxyethyl starch thus obtained is purified and

   g) spray-dried,

   characterised in that
   2-chloroethanol is used as hydroxyethylation agent and hydroxyethylation is carried out under alkaline conditions at room temperature.

4. Process according to claim 3, characterised in that the pH value is kept at a value of about 12 during hydroxyethylation.

5. Process according to claim 3 or claim 4, characterised in that the temperature is kept at a value of about 20 to 25°C.

6. Process according to any one of claims 3 to 5, characterised in that the hydroxyethyl starch is purified by filtration and ultrafiltration.

**Revendications**

**Revendications pour les Etats contractants suivants : AT, BE, CH, LI, DE, FR, GB, GR, IT, LU, NL, SE**

1. Hydroxyéthylamidon à mettre en oeuvre comme expanseur du plasma, que l'on obtient par dégradation hydrolytique préalable d'un amidon riche en amylopectine, par hydroxyéthylation partielle jusqu'à ce que l'on obtienne un degré de substitution déterminé en présence d'alcalis et par dégradation hydrolytique ultérieure pour obtenir un poids moléculaire déterminé, caractérisé en ce que

   il présente un poids moléculaire moyen $M_w$ de 80.000 à 300.000 et un degré de substitution MS de 0,2 à 0,4,

   le rapport de la substitution en $C_2$ à la substitution en $C_6$ des unités d'anhydroglucose s'élève de 8 à 20, et

   le degré de substitution DS se situe dans le domaine de 0,15 à 0,40.

2. Hydroxyéthylamidon selon la revendication 1, caractérisé en ce qu'il présente un poids moléculaire moyen $M_w$ de 100.000 à 300.000 et un degré de substitution MS de 0,25 à 0,35, le rapport de la substitution en $C_2$ à la substitution en $C_6$ des unités

d'anhydroglucose s'élève de 8 à 20 et le degré de substitution DS se situe dans le domaine de 0,2 à 0,35.

3. Procédé pour la préparation d'hydroxyéthylamidon selon la revendication 1, dans lequel

a) on soumet l'amidon qui présente une teneur en amylopectine > 95 % à une extraction préalable dans du méthanol,

b) on amène l'amidon par hydrolyse acide à un poids moléculaire moyen déterminé,

c) on soumet l'amidon à un lavage alcalin,

d) on soumet l'amidon à une hydroxyéthylation à l'aide d'un agent d'hydroxyéthylation dans des conditions alcalines,

e) on règle précisément le poids moléculaire par hydrolyse acide,

f) on purifie l'hydroxyéthylamidon ainsi obtenu, et

g) on le sèche par pulvérisation,

caractérisé en ce qu'on utilise, à titre d'agent d'hydroxyéthylation, du 2-chloréthanol et on effectue l'hydroxyéthylation dans des conditions alcalines à la température ambiante.

4. Procédé selon la revendication 3, caractérisé en ce qu'on maintient le pH à une valeur d'environ 12 au cours de l'hydroxyéthylation.

5. Procédé selon la revendication 3 ou 4, caractérisé en ce qu'on maintient la température à une valeur d'environ 20 à 25°C.

6. Procédé selon l'une quelconque des revendications 3 à 5, caractérisé en ce qu'on purifie l'hydroxyéthylamidon par filtration et par ultrafiltration.

7. Utilisation d'hydroxyéthylamidon conformément à au moins une des revendications précédentes dans la préparation d'un succédané colloïdal du plasma.

**Revendications pour l'Etat contractant suivant : ES**

1. Utilisation d'hydroxyéthylamidon, que l'on obtient par dégradation hydrolytique préalable d'un amidon riche en amylopectine, par hydroxyéthylation partielle jusqu'à ce que l'on obtienne un degré de substitution déterminé en présence d'alcalis et par dégradation hydrolytique ultérieure pour obtenir un poids moléculaire déterminé, dans la préparation d'un succédané colloïdal du plasma dans laquelle l'hydroxyéthylamidon présente un poids moléculaire moyen $M_w$ de 80.000 à 300.000 et un degré de substitution MS de 0,2 à 0,4, le rapport de la substitution en $C_2$ à la substitution en $C_6$ des unités d'anhydroglucose s'élève de 8 à 20, et le degré de substitution DS se situe dans le domaine de 0,15 à 0,40.

2. Utilisation selon la revendication 1, caractérisée en ce que l'hydroxyéthylamidon présente un poids moléculaire moyen $M_w$ de 100.000 à 300.000 et un degré de substitution MS de 0,25 à 0,35, le rapport de la substitution en $C_2$ à la substitution en $C_6$ des unités d'anhydroglucose s'élève de 8 à 20 et le degré de substitution DS se situe dans le domaine de 0,2 à 0,35.

3. Procédé pour la préparation d'hydroxyéthylamidon selon la revendication 1 ou 2, dans lequel

a) on soumet l'amidon qui présente une teneur en amylopectine > 95 % à une extraction préalable dans du méthanol,

b) on amène l'amidon par hydrolyse acide à un poids moléculaire moyen déterminé,

c) on soumet l'amidon à un lavage alcalin,

d) on soumet l'amidon à une hydroxyéthylation à l'aide d'un agent d'hydroxyéthylation dans des conditions alcalines,

e) on règle précisément le poids moléculaire par hydrolyse acide,

f) on purifie l'hydroxyéthylamidon ainsi obtenu, et

g) on le sèche par pulvérisation,

caractérisé en ce qu'on utilise, à titre d'agent d'hydroxyéthylation, du 2-chloréthanol et on effectue l'hydroxyéthylation dans des conditions alcalines à la température ambiante.

4. Procédé selon la revendication 3, caractérisé en ce qu'on maintient le pH à une valeur d'environ 12 au cours de l'hydroxyéthylation.

5. Procédé selon la revendication 3 ou 4, caractérisé en ce qu'on maintient la température à une valeur d'environ 20 à 25°C.

6. Procédé selon l'une quelconque des revendications 3 à 5, caractérisé en ce qu'on purifie l'hydroxyéthylamidon par filtration et par ultrafiltration.